# EUROPEAN PATENT APPLICATION

(11) **EP 4 008 395 A1**
(43) Date of publication of application: **08.06.2022**
(21) Application number: 20850817.6
(22) Date of filing: 03.08.2020
(51) Int. Cl.: A61N 1/36, A61B 5/053

(54) **BIOLOGICAL MEASUREMENT SYSTEM, BIOLOGICAL MEASUREMENT PROGRAM, AND COMPUTER-READABLE NON-TRANSITORY STORAGE MEDIUM**

(30) Priority: 02.08.2019 JP 2019142941
(71) Applicant: Tanita Corporation, Tokyo 174-8630 (JP)
(72) Inventor: SHIMIZU Tomoka, Tokyo 174-8630 (JP); KASAHARA Yasuhiro, Tokyo 174-8630 (JP); TSUTAYA Takao, Tokyo 174-8630 (JP)
(74) Representative: Eisenführ Speiser
(86) International application number: PCT/JP2020/029629
(87) International publication number: WO 2021/024973

(57) **Abstract**

A biometric measurement system capable of measuring an immediate response of a living body to the intervention of a physical stimulus to the living body is provided. A biometric measurement system (210) capable of applying electrical muscle stimulation to a living body comprises: an electrode for bioelectrical impedance measurement (217a-217d) for measuring a bioelectrical impedance of a living body; and an electrode for electrical muscle stimulation (217e, 217f) for applying electrical muscle stimulation to the living body, wherein the electrode for bioelectrical impedance measurement (217a-217d) and the electrode for electrical muscle stimulation (217e, 217f) are arranged to contact the living body simultaneously.

## Description

### CROSS-REFERENCE TO RELATED APPLICATIONS

This application claims the benefit of Patent Application No. 2019-142941 filed in Japan on August 2, 2019, the contents of which application are hereby incorporated by reference.

### TECHNICAL FIELD

The present disclosure relates to a biometric measurement system, a biometric measurement program, and a computer-readable non-transitory storage medium recording the program, each for measuring responses of a living body to an intervention of a physical stimulus to the living body.

### BACKGROUND TECHNOLOGY

When a living body is subjected to physical stimulation (electrical stimulation) by an electric muscle stimulation (EMS) device, etc., and passive muscle contraction movements are performed, or when the body is subjected to external physical stimulation (mechanical stimulation) such as massage, etc., changes such as relaxation of stiffness and improvement of blood circulation occur in the body. Physical stimulation is also given to the muscles by the body's own active exercise, which in turn causes changes in the body.

There is a system that accumulates the changes in the body when physical stimulation is given to the body and evaluates the long-term changes of the body over time. For example, JP2007075586A discloses a health management system that feeds back health management information based on past biological information. For example, JP3948617B2 discloses a device that measures the muscle tone diagram of a subject's muscles when the subject exercises against a load and determines the ratio of the type of muscle that the subject's measurement area has from the time series data of the measured muscle tone diagram. In addition, for example, JP4990719B2 discloses a health measurement device that evaluates exercise function based on the center of gravity sway and at the same time measures body composition using bioelectric impedance analysis (BIA) to comprehensively evaluate biological activity function.

### SUMMARY OF THE INVENTION

The conventional system or device can evaluate relatively long-term changes in a living body over time based on past accumulated information, but it cannot measure the immediate response of a living body to the intervention of physical stimuli.

Therefore, one of the purposes of the present disclosure is to provide a biometric measurement system that can measure the immediate response of a living body due to the intervention of a physical stimulus to the living body.

The biometric measurement system of one aspect of the present proposals has the following structure:

(1) A biometric measurement system capable of providing electrical muscle stimulation to a living body, comprising:
   an electrode for bioelectrical impedance measurement for measuring a bioelectrical impedance of a living body, and
   an electrode for electrical muscle stimulation for applying electrical muscle stimulation to the living body, wherein
   the bioelectrical impedance measurement electrode and the electrical muscle stimulation electrode are arranged to contact the living body simultaneously.
(2) The biometric measurement system according to (1), wherein the electrode for bioelectrical impedance measurement and the electrode for electrical muscle stimulation are disposed apart from each other.
(3) The biometric measurement system according to (2), wherein the electrode for bioelectrical impedance measurement and the electrode for electrical muscle stimulation are arranged to contact a sole of a foot of the living body simultaneously.
(4) The biometric measurement system according to any one of (1) to (3), comprising:
   a plurality of said electrodes for bioelectrical impedance measurement for a left portion;
   a plurality of said electrodes for bioelectrical impedance measurement electrodes for a right portion;
   at least one said electrode for electrical muscle stimulation for a left portion; and
   at least one said electrode for electrical muscle stimulation for a right portion.
5. The biometric measurement system according to (4), comprising:
   the electrode for bioelectrical impedance measurement of the toe side for the left foot;
   the electrode for bioelectrical impedance measurement of the heel side for the left foot;
   the electrode for electrical muscle stimulation of a central portion for the left foot;
   the electrode for bioelectrical impedance measurement of the toe side for the right foot;
   the electrode for bioelectrical impedance measurement of the heel side for the right foot; and
   the electrode for electrical muscle stimulation of a central portion for the right foot.
(6) The biometric measurement system according to (5), wherein the electrodes for electrical muscle stimulation have a raised shape that fits the shape of the central portion.
(7) The biometric measurement system according to (5), wherein the electrodes for electrical muscle stimulation are raised and can be pushed in.
(8) The biometric measurement system according to (4), comprising:
   the electrode for bioelectrical impedance measurement of the toe side for the left foot;
   the electrode for bioelectrical impedance measurement of the heel side for the left foot;
   the electrode for electrical muscle stimulation of the toe side for the left foot;
   the electrode for electrical muscle stimulation of the heel side for the left foot;
   the electrode for bioelectrical impedance measurement of the toe side for the right foot;
   the electrode for bioelectrical impedance measurement of the heel side for the right foot; and
   the electrode for electrical muscle stimulation of the toe side for the right foot; and
   the electrode for electrical muscle stimulation on the heel side for the right foot.
(9) The biometric measurement system according to any one of (1) to (8), wherein the electrode for bioelectrical impedance measurement and the electrode for electrical muscle stimulation are made of different materials.
(10) The biometric measurement system according to any one of (1) to (9), further comprising an output unit that outputs information based on the bioelectrical impedance measured using the electrode for the bioelectrical impedance measurement in association with the intervention by the electrical muscle stimulation using the electrodes for electrical muscle stimulation.

A biometric measurement system in one aspect, comprises: an intervention determination unit for determining contents of intervention of physical stimulation to a living body; a biometric measuring unit for measuring biometric information of the living body; and an output unit that outputs the biometric information measured by the biometric measurement unit or information based on the biometric information associated with the intervention determined by the intervention determination unit.

A biometric measurement system in one aspect comprises: an intervention determination unit that determines contents of intervention of physical stimulation to a living body; a body composition calculating unit that calculates a body composition of the living body; and a memory unit that stores the body composition and the contents of the intervention in association with each other.

### BRIEF DESCRIPTION OF THE DRAWINGS

Fig. 1A shows an external view of a biometric measurement system (front side) of the first embodiment;
Fig. 1B shows an external view of the biometric measurement system (back side) of the first embodiment;
Fig. 2 shows a situation in which the biometric measurement system of the first embodiment is attached to the human body as a living body;
Fig. 3 shows a block diagram of the biometric measurement system of the first embodiment;
Fig. 4 shows a relationship between the amount of intervention and the change in bioelectrical impedance before and after the intervention in the first embodiment;
Fig. 5 shows an example of a display of a display panel in the first embodiment;
Fig. 6 shows a table showing a relationship between the amount of water content in a living body and bioelectrical impedance, etc. in the first embodiment;
Fig. 7 shows a table showing the relationship between changes in the amount of water content of a living body and changes in bioelectrical impedance, etc. in the first embodiment;
Fig. 8 shows an example of recording of body composition values in the first embodiment;
Fig. 9 shows a flowchart of the biometric measurement method in the biometric measurement system of the first embodiment;
Fig. 10 shows a biometric measurement system of Variant 1 of the first embodiment;
Fig. 11 shows a block diagram of the biometric measurement system of Variant 1 of the first embodiment;
Fig. 12 shows an external view of a biometric measurement system of the second embodiment;
Fig. 13 shows the first variation of the electrode arrangement of the second embodiment;
Fig. 14 shows the second variation of the electrode arrangement of the second embodiment;
Fig. 15 shows the third variation of the electrode arrangement of the second embodiment;
Fig. 16 shows the fourth variation of the electrode arrangement of the second embodiment; and
Fig. 17 shows the fifth variation of the electrode arrangement of the second embodiment.

### DETAILED DESCRIPTION OF THE INVENTION

The following is a description of the embodiments of the present disclosure with reference to the drawings. The embodiments below show examples of implementing the present invention, and do not limit the present invention to the specific configuration described below. In the implementation of the present invention, the specific configurations according to the embodiments may be adopted as appropriate.

### (First embodiment)

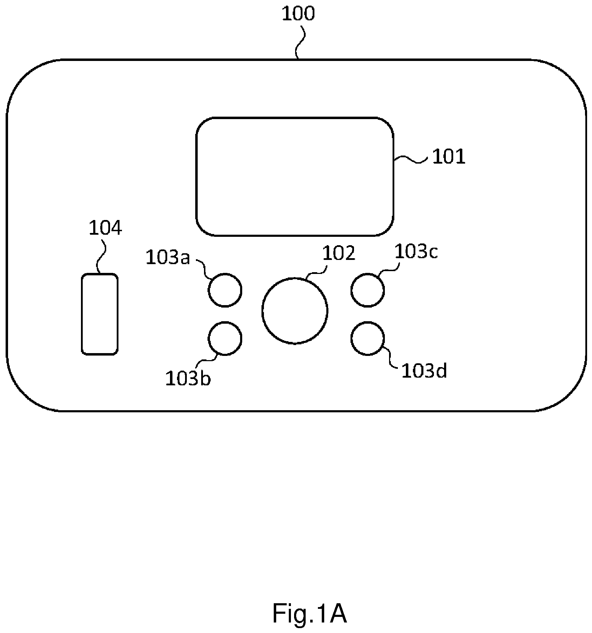

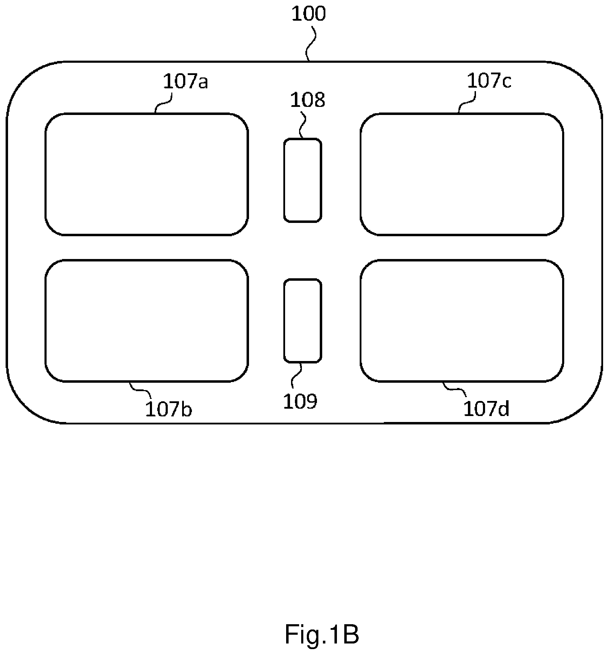

Fig. 1 is an external view of a biometric measurement system of the first embodiment. The biometric measurement system 100 is configured as a single pad-like device. The biometric measurement system 100 is used in contact with the surface of a living body. Fig. 1A shows the surface (front side) that becomes the outside when the biometric measurement system 100 is placed in contact with a living body, and Fig. 1B shows the surface (back side) of the biometric measurement system 100 that contacts the living body.

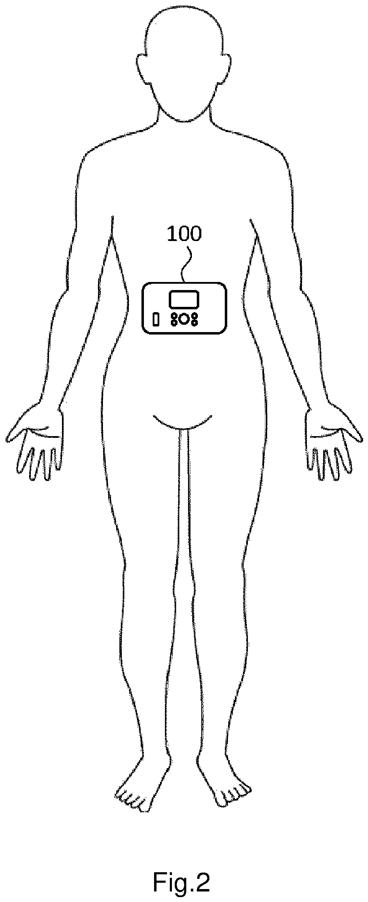

Fig. 2 shows the biometric measurement system mounted on a human body, a living body. As shown in Fig. 2, the biometric measurement system 100 is designed to be worn on the abdomen of a human body. The biometric measurement system 100 may be equipped with a fixing aid such as a belt to fix it in contact with the human body.

As shown in Fig. 1A, the biometric measurement system 100 has a display panel 101, a start button 102, four operation buttons 103a to 103d (hereinafter collectively referred to as "operation buttons 103"), and an environmental thermometer 104. In addition, as shown in Fig. 1B, the biometric measurement system 100 has four electrodes 107a to 107d (hereinafter collectively referred to as "electrodes 107"), a body surface thermometer 108, and a body surface hardness tester 109. The display panel 101 corresponds to an output unit.

The biometric measurement system 100 functions as an intervention device (specifically, an EMS device) causes the living body to perform passive movements by reflexively contracting the muscles of the living body by applying electrical stimulation (hereinafter also referred to as "electrical muscle stimulation") to the living body through the electrodes 107, and also functions as a biometric device (specifically, a four-electrode BI body composition analyzer) that measures the bioelectrical impedance of the living body by sending and receiving a weak electric current through the electrodes 107 using the same electrodes 107. The passive exercise by electrical muscle stimulation and the body composition measurement by BI method cannot be performed at the same time, so they are performed at different times.

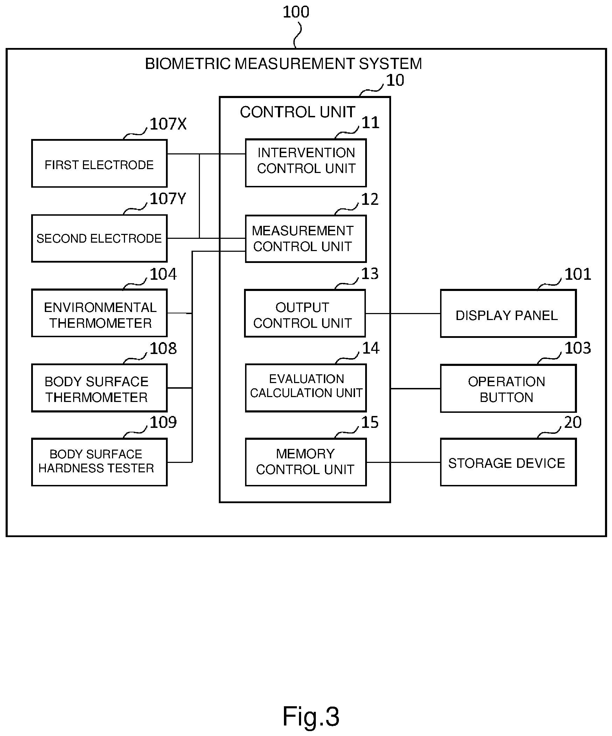

Fig. 3 is a block diagram of the biometric measurement system. As shown in Fig. 3, the biometric measurement system 100 includes a first electrode 107X, a second electrode 107Y, an environmental thermometer 104, the body surface thermometer 108, the body surface hardness tester 109, a control device 10, a display panel 101, operation buttons 103, and a memory device 20. In this system, the electrodes 107a and 107c of Fig. 1 are used as the first electrode 107X, and electrodes 107b and 107d correspond to the second electrode 107Y. The first electrode 107X is used as a current-carrying electrode when the biometric measurement system 100 functions as a body composition analyzer, and the second electrode 107Y functions as a measurement electrode when the biometric measurement system 100 functions as a body composition analyzer.

The control device 10 includes an intervention control unit 11, a measurement control unit 12, an output control unit 13, an evaluation calculation unit 14, and a memory control unit 15. The control device 10 may be realized by a general-purpose processor executing a predetermined computer program, or may be configured by a dedicated circuit. The computer program may be provided externally via a non-transitory recording medium or communication and stored in the biometric measurement system 100, or it may be stored in the biometric measurement system 100 in advance.

The intervention control unit 11 controls the biometric measurement system 100 to function as an EMS device. The intervention control unit 11 performs control to make the biometric measurement system 100 functions as an EMS device. Specifically, the intervention control unit 11 controls the first electrode 107X and the second electrode 107Y to provide electrical stimulation to the living body in a predetermined pattern and intensity according to the intervention program, thereby causing the living body to perform a passive exercise.

The measurement control unit 12 controls the biometric measurement system 100 to function as a body composition analyzer and obtains biometric information of the living body. Specifically, the measurement control unit 12 measures bioelectrical impedance as biometric information by applying weak AC currents of high and low frequencies to the living body from the first electrode 107X and measuring the current received by the second electrode 107Y. In addition, biometric information such as the user's height, weight, age, gender, etc., is given to the measurement control unit 12 using the operation button 103, for example, and by combining the biometric information with the bioelectrical impedance measured using the first electrode 107X and the second electrode 107Y, the body composition of the user, such as body fat percentage, muscle mass, and water content, is obtained. In addition, the measurement control unit 12 may read out the biometric information of the user, such as the user's height, weight, age, and gender, stored in advance, and such biometric information may be provided to the measurement control unit 12.

The measurement control unit 12 controls the first electrode 107X and the second electrode 107Y to obtain bioelectrical impedance as biometric information in association with the intervention by the intervention control unit 11, and in addition, controls the body surface thermometer 108 and the body surface hardness tester 109 to obtain the body surface temperature and the body surface hardness as biometric information in association with the intervention by the intervention control unit 11. Furthermore, the measurement control unit 12 controls the environmental thermometer 104 to obtain the environmental temperature as environmental information in association with the intervention by the intervention control unit 11.

A plurality of intervention programs are stored in the intervention control unit 11. The intervention program is a procedure in which multiple types of electrical stimulation patterns are executed in order according to a predetermined sequence. The intervention control unit 11 determines the intervention contents and then performs the intervention. The intervention control unit 11 automatically determines the intervention program based on the biometric and environmental information measured before determining the intervention content by the measurement control unit 12 (intervention program automatically determining mode).

In the intervention program automatically determining mode, the user can set a goal. Specifically, the user can designate any item of body composition as a target item and set a target value for that target item. In this goal-setting, the user can also set the deadline for achieving the goal or the target number of days to achieve the goal. In the intervention program automatically determining mode, when a goal is set, the intervention control unit 11 determines the intervention program based on the set goal so that the target value is reached within the deadline for achieving the goal from the current body composition value.

In the intervention program automatically determining mode, the intervention program can be determined automatically based on the measured biometric information even when no goal is set. For example, the intervention control unit 11 may compare each item of the body composition measured by the biometric measurement unit 12 with a standard value and determine an intervention program so that the item with a large deviation from the standard value approaches the standard value.

When the user specifies the intervention contents by operating the operation button 103, the intervention control unit 11 determines the intervention contents according to the specification. When the user specifies the intervention content, the user can select and specify any of the intervention programs (intervention program-specified mode) or can select and specify any of a plurality of prepared patterns of electrical stimulation (pattern-specified mode). The user can specify the intensity and duration of the electrical stimulation in either mode. In the intervention program automatically determining mode, the intensity and duration may also be set automatically.

The intervention control unit 11 determines the content of the electrical muscle stimulation based on the inputted biometric information such as height, weight, age, gender, body composition, and input from the user. For example, since the stimulus per unit amount differs depending on the muscle mass even when the same current is applied, the intervention control unit 11 sets the stimulus in accordance with the muscle mass. In addition, the intervention control unit 11 adjusts the input signal according to the amount of subcutaneous fat because a large amount of subcutaneous fat increases the contact impedance and makes it easier to feel the stimulus.

The output control unit 13 displays a guidance display on the display panel 101 to determine the intervention contents. When the user operates the operation button 103 to select one of the modes, the intervention control unit 11 determines the intervention contents automatically or according to the user's operation. When the user operates the operation button 103 and gives the start instruction, the intervention control unit 11 starts the intervention of electrical muscle stimulation.

The measurement control unit 12 measures the bioelectrical impedance before the intervention control unit 11 starts the intervention. The bioelectrical impedance measured at this time is before the start of the intervention and is referred to as the "pre-intervention bioelectrical impedance." The measurement control unit 12 also measures the bioelectrical impedance at predetermined timing during the intervention, that is, at a timing when the electrical muscle stimulation for passive exercise is not provided. This bioelectrical impedance is in the middle of the intervention and is referred to as "in-process bioelectrical impedance." Furthermore, the measurement control unit 12 measures the bioelectrical impedance at the timing when the intervention program ends. This bioelectrical impedance is the one after the intervention is completed and is referred to as "post-intervention bioelectrical impedance."

The measurement control unit 12 similarly controls the environmental thermometer 104, body surface thermometer 108, and body surface hardness tester 109 to measure the environmental temperature, body surface temperature, and body surface hardness, respectively, before the intervention, at predetermined timing during the intervention (e.g., when the bioelectrical impedance is measured), and after the intervention. The environmental temperature may be measured only before the intervention. The biometric information such as bioelectrical impedance, body surface temperature, and body surface hardness before, during, and after the intervention are referred to as "pre-intervention biometric information," "in-process biometric information," and "post-intervention biometric information," respectively.

The evaluation calculation unit 14 calculates the evaluation of the living body based on the progress of the intervention program by the intervention control unit 11 and the results of biometric measurements performed at corresponding times. The evaluation calculation unit 14 may output the measured biometric information as it is, or may visualize (e.g., graph) the measured biometric information so that it can be evaluated, or may perform a predetermined calculation based on the biometric data to calculate an evaluation value different from the biometric data itself. The output control unit 13 displays the evaluation results obtained by the evaluation calculation unit 14 on the display panel 101.

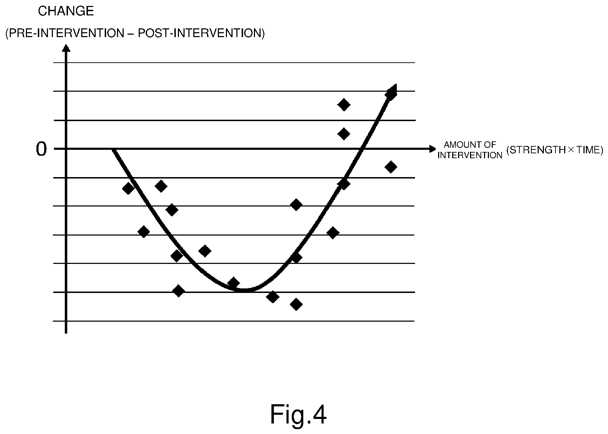

Fig. 4 is a graph showing the relationship between the amount of intervention and the change in bioelectrical impedance before and after the intervention. The amount of intervention is defined as the amount obtained by integrating the intensity of the electrical muscle stimulation with the intervention time in this embodiment, but other definitions may be used. As shown in the graph in Fig. 4, when the amount of intervention is relatively small, the change in bioelectrical impedance (pre-intervention bioelectrical impedance - post-intervention bioelectrical impedance) increases in the negative direction as the amount of intervention increases. This indicates that as the amount of intervention increases, the amount of water content in the intervened area decreases as a response of the organism, and this can be evaluated as a resolution of edema. In addition, when the amount of intervention is above a certain level, the bioelectrical impedance increases in a positive direction as the amount of intervention increases. This can be evaluated as the edema being resolved (the decrease in water content due to the resolution of edema is ceased) and the increase in blood flow due to the intervention.

In this way, the response of a living body can be evaluated by the change in bioelectrical impedance, but if body composition, body surface temperature, and body surface hardness are also taken into account, the response of a living body can be estimated more accurately. In the above example, the resolution of edema and the increase in blood flow can be estimated and evaluated by changes in bioelectrical impedance, but if body surface temperature and/or body surface hardness are also taken into account, the resolution of edema and the increase in blood flow can be separated and estimated more accurately.

Therefore, the evaluation calculation unit 14 distinguishes between the change in water content due to the resolution of edema and the change in water content due to the increase in blood flow, also taking into account the body temperature measured by the body surface thermometer 108. In other words, when the change in body surface temperature is relatively small, edema can be evaluated as being resolved, although there is no increase in blood flow, and when the body surface temperature is increased, blood flow can be evaluated as being increased. Therefore, by considering the change in water content and the change in body surface temperature at the same time, it is possible to determine whether the change in water content is due to the resolution of edema, the increase in blood flow, or both.

In addition, as muscle mass increases, sensitivity to electrical muscle stimulation decreases. Therefore, the more muscle mass there is, the higher the amount of intervention required to turn from a state of edema resolution to a state of increased blood flow tends to be. Therefore, the evaluation calculation unit 14 distinguishes between the change in water content due to the resolution of edema and the change in water content due to the increase in blood flow, taking into account the body composition values (specifically, muscle mass). In other words, when the muscle mass is relatively large, the evaluation calculation unit 14 sets a relatively large amount of intervention to change from the state of edema resolution to the state of increased blood flow and determines whether the obtained bioelectrical impedance change is in the state of edema resolution or in the state of increased blood flow.

The evaluation calculation unit 14 evaluates "edema is resolved," "blood flow is increased," etc. based on the measured bioelectrical impedance before and after the intervention, changes in body surface temperature, and body composition values. The output control unit 13 displays the result of this evaluation in textual information on the display panel 101. This allows the user to obtain information such as "edema is resolved," "blood flow is increased," etc. as a response of the living body to the intervention.

In the case where the bioelectrical impedance is measured while the intervention program is progressing, the change from the bioelectrical impedance before the intervention to the bioelectrical impedance during the intervention will change in the same way as the graph shown in Fig. 4 as the intervention program progresses. Therefore, while the intervention program is in progress, the evaluation calculation unit 14 may evaluate the response of the living body each time, and the output control unit 13 may display the evaluation results in real-time. In this case, the display panel 101 displays the response of the living body in real-time as the intervention program progresses, such as "edema is being resolved," "edema has been resolved," "blood flow is being increased," etc.

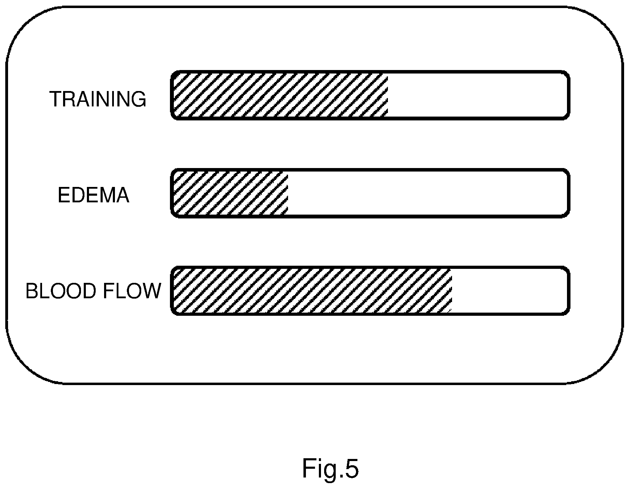

Fig. 5 shows an example of a display of the display panel. In the example of Fig. 5, the evaluation calculation unit 14 evaluates the degree of the resolution of edema and the degree of increase in blood flow. The output control unit 13 displays the degree of resolution of edema, and the degree of increase in blood flow evaluated by the evaluation calculation unit 14 are displayed on the display panel 101 in the form of an indicator in association with the degree of progress of the intervention program (intervention amount).

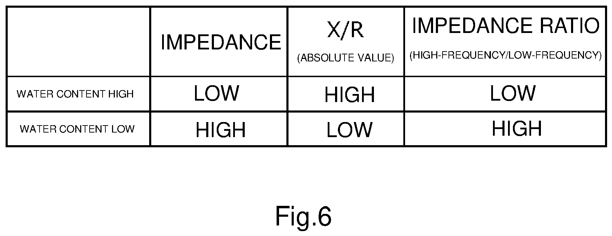

Fig. 6 is a table showing the relationship between the amount of water content in the living body and the bioelectrical impedance, etc. As shown in Fig. 6, when the water content of the living body is high, the bioelectrical impedance becomes low, the absolute value of the reactance/resistance ratio (X/R) becomes high, and the high-frequency impedance/low-frequency impedance ratio becomes lower. On the other hand, when the amount of water content in the body is low, the bioelectrical impedance becomes high, the absolute value of the reactance/resistance ratio (X/R) becomes low, and the ratio of high-frequency impedance to low-frequency impedance becomes high.

Therefore, the evaluation calculation unit 14 estimates the water content of the living body based on the bioelectrical impedance, the absolute value of the ratio of reactance/resistance (X/R), and the ratio of high-frequency impedance/low-frequency impedance according to the table in Fig. 6.

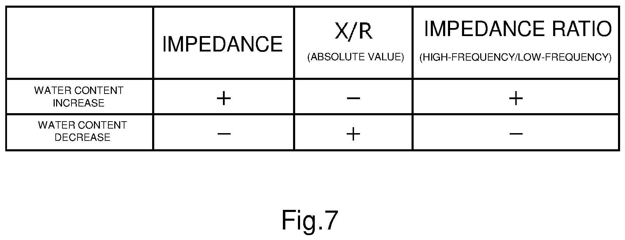

Fig. 7 is a table showing the relationship between changes in the water content of a living body and changes in bioelectrical impedance and other parameters. As shown in Fig. 7, when the water content of the living body increases, the bioelectrical impedance increases, the absolute value of the reactance/resistance ratio (X/R) decreases, and the ratio of high-frequency impedance to low-frequency impedance increases. On the other hand, when the amount of water content in the living body decreases, the bioelectrical impedance decreases, the absolute value of the reactance/resistance ratio (X/R) increases, and the ratio of high-frequency impedance to low-frequency impedance decreases.

Therefore, the evaluation calculation unit 14 estimates the change (increase or decrease) in the water content of the living body according to the table in Fig. 7 based on the bioelectrical impedance, the absolute value of the ratio of reactance/resistance (X/R), and the change (increase or decrease) in the ratio of high-frequency impedance/low-frequency impedance.

When the intervention is performed in the intervention program automatically determining mode and the bioelectrical impedance is measured in the middle of the program, the intervention control unit 11 dynamically changes the content of the subsequent intervention according to the result of the measurement of the bioelectrical impedance in the middle of the program. For example, the intervention control unit 11 may terminate the intervention when the intermediate bioelectrical impedance reaches a predetermined state. For example, the intervention control unit 11 may increase the intensity of the electrical muscle stimulation when there is no significant change in the in-process bioelectrical impedance. Alternatively, the intervention control unit 11 may determine the pattern of the subsequent electrical muscle stimulation according to the in-process bioelectrical impedance.

The intervention control unit 11 may change the content of the subsequent intervention based on the midway bioelectrical impedance in the same way in the intervention program-specified mode or pattern-specified mode. In this case, the intervention control unit 11 may dynamically change the intensity and duration of the electrical muscle stimulation based on the midway bioelectrical impedance.

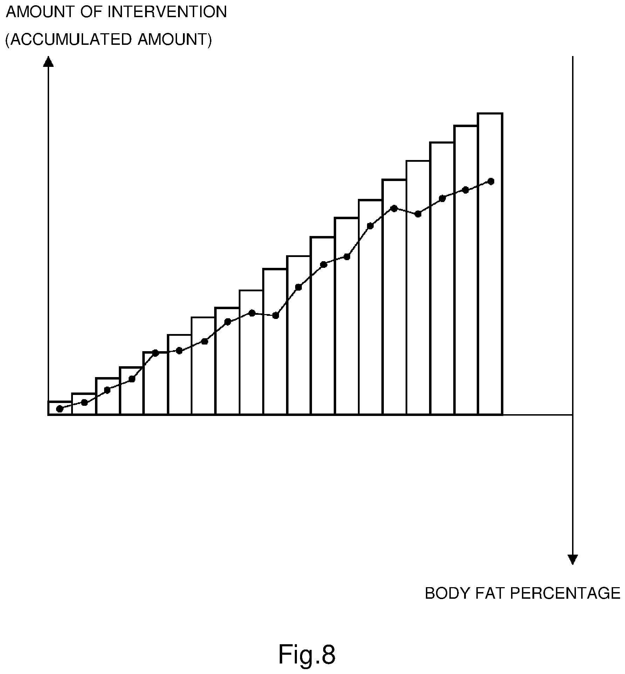

Fig. 8 is a graph showing an example of a recording of body composition values. As described above, the biometric system can obtain an immediate response of the body to the intervention, i.e., an immediate change in bioelectrical impedance, body surface temperature, body surface hardness, etc., or immediate effects of the intervention based on such changes by evaluating the biometric information before the intervention and the biometric information during and after the intervention in relation to the intervention. On the other hand, repeated interventions over a long period of time (e.g., weeks to months) will result in gradual changes in body composition (e.g., muscle mass, body fat percentage, etc.).

Therefore, in the biometric measurement system 100, the memory control unit 15 stores, for each intervention performed by the intervention control unit 11, the In the biometric measurement system 100, the memory control unit 15 stores in the memory device 20 the body composition value at the time of the intervention and the intervention content or the intervention amount of the intervention. The amount of intervention and body composition values of past interventions are accumulated in the storage device 20. Fig. 8 shows the accumulated amount of intervention in a bar graph and the body fat percentage, an item of body composition values, in a line graph. In the example of Fig. 8, it is shown that the body fat percentage is decreasing as the cumulative amount of intervention increases. The output control unit 13 displays the gradual changes in biological information due to the intervention, as shown in Fig. 8 in numerical values and graphs on the display panel 13.

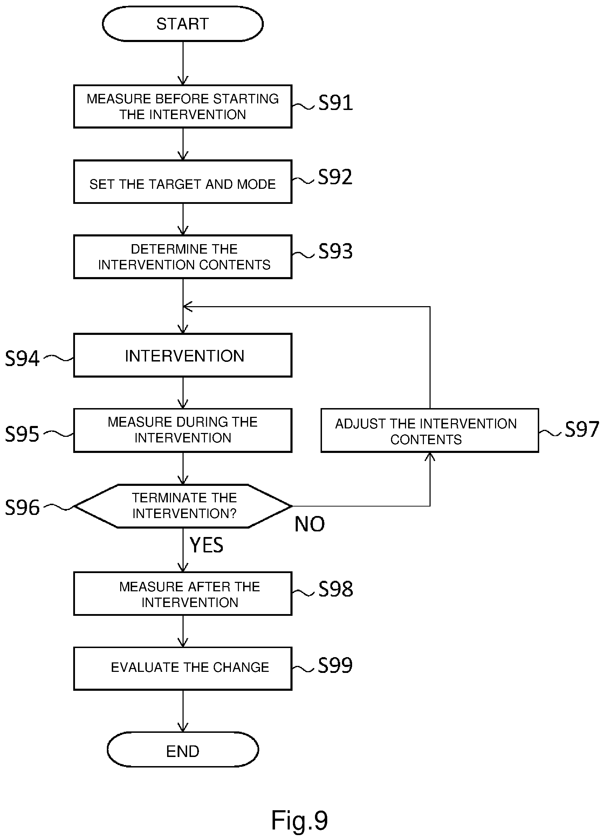

Fig. 9 shows a flowchart of a biometric measurement method in the biometric measurement system. The measurement control unit 12 first, before starting the intervention, measures the pre-intervention biometric information, i.e., the bioelectrical impedance, body surface temperature, and body surface hardness before the start of the intervention using the electrodes 107, the body surface thermometer 108, and the body surface hardness tester 109 (Step S91). At this time, the measurement control unit 12 also measures the environmental temperature using the environmental thermometer 104.

The user sets the target and mode by operating the operation button 103 (Step S92). The user sets an item of body composition, a body composition value, and a deadline for achieving the target as target by operating the operation button 103. The user also selects one of the above-mentioned intervention programs automatically determining mode, the intervention program-specified mode, or the pattern-specified mode as the mode.

The intervention control unit 11 determines the intervention contents according to the mode setting by the user (Step S93). If the user sets the mode for the intervention program automatically determining mode, the intervention control unit 11 determines the intervention program and its intensity based on the user's body composition and goals. If the user sets the intervention program-specified mode, the intervention control unit 11 determines the intervention program and intensity specified by the user as the intervention content. In the case where the user sets the pattern-specified mode, the intervention control unit 11 determines the pattern and intensity specified by the user as the intervention contents. At this time, the user also sets the duration of the intervention.

The intervention control unit 11 starts the intervention in response to the operation of the start button 102 (step S94). The intervention control unit 11 pauses the intervention in the middle of the intervention, and the biometric measurement unit 12 measures the biometric information during the intervention (Step S95).

The intervention control unit 11 determines whether or not to terminate the intervention (step S96). If the intervention is to be terminated according to the biometric information measured in the middle, the intervention control unit 11 terminates the intervention when the evaluation calculation unit 14 evaluates that the desired effect has been obtained according to the biometric information in the middle. Alternatively, the intervention control unit 11 terminates the intervention when the set time has elapsed.

When the intervention control unit 11 does not terminate the intervention (NO in step S96), it adjusts the intervention contents according to the bioelectrical impedance, etc. (step S97) and continues the intervention (step S94). When the intervention control unit 11 finishes the intervention (YES in step S96), the biometric measurement unit 12 measures the biometric information after the intervention (step S98).

The evaluation calculation unit 14 evaluates the change from the biometric data before the intervention to the biometric data after the intervention (Step S99). At this time, the output control unit 13 displays the results of the evaluation obtained by the evaluation calculation unit 14 on the display panel 103. The memory control unit 15 stores the evaluation obtained by the evaluation calculation unit 14 together with the body composition values measured by the biometric unit 12 in the memory device 20.

As described above, the biometric measurement system 100 of the present embodiment evaluates changes in biometric information caused by passive exercise by intervening electrical muscle stimulation and measuring biometric information. In this way, the immediate response of the body to passive exercise can be determined. In addition, by measuring the biometric information during the passive exercise, the response of the body in real-time during the passive exercise can be known, and the content of the subsequent intervention of electrical muscle stimulation can be dynamically adjusted.

### (Variant 1)

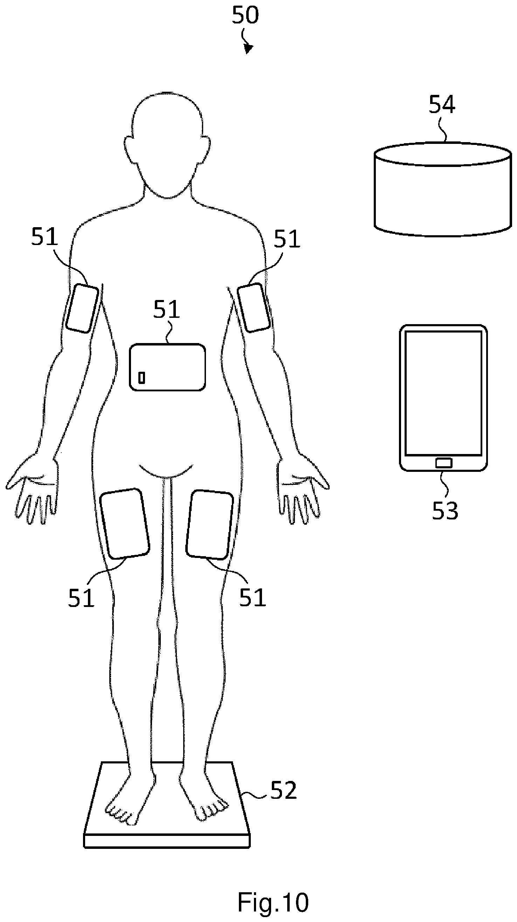

Fig. 10 shows the biometric measurement system of Variant 1. In the above embodiment, the biometric measurement system 100 was configured as a single pad-like device, but in this variation, the biometric measurement system 50 is composed of a plurality of EMS devices 51 as intervention devices, a body composition analyzer 52 as a biometric device, an information terminal device 53 (e.g., smartphone or tablet PC) as a control measure, and a storage device 54.

The plural EMS devices 51 are used by attaching them to the abdomen, both arms, and both legs of the human body, respectively. In other words, a plurality of EMS devices 51 are used for a plurality of intervention portions on the human body. The body composition analyzer 52 is a flat-plate device with four electrodes on its top surface. The four electrodes are designed so that when a person stands on the body composition analyzer 52, the soles of each left and right foot come into contact with two electrodes each. The information terminal device 53 has the function of communicating wirelessly or wired with the EMS device 51, the body composition analyzer 52, the information terminal device 53, and the storage device 54. The storage device 54 is provided on a communication network and transmits and receives information through network communication with the information terminal device 53.

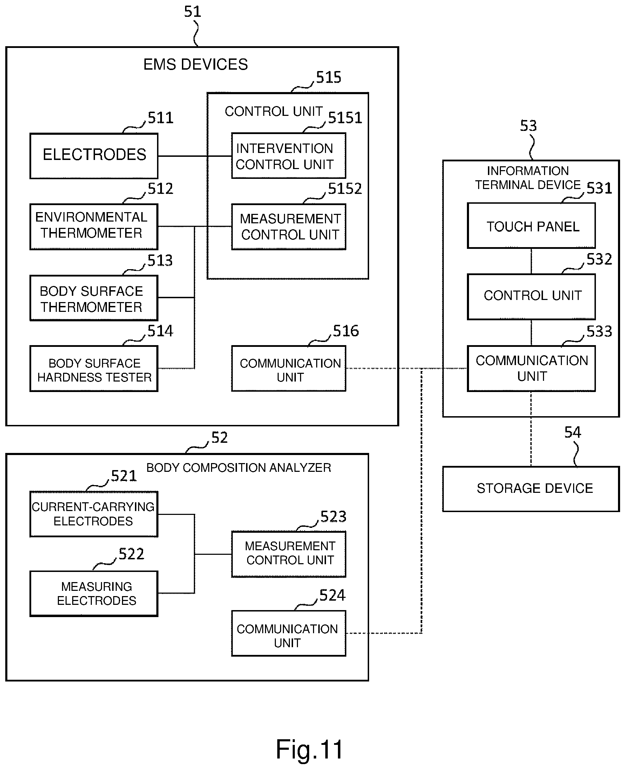

Fig. 11 shows a block diagram of the configuration of the biometric measurement system of Variant 1. The EMS device 51 has electrodes 511, an environmental thermometer 512, a body surface thermometer 513, a body surface hardness tester 514, a control unit 515, and a communication unit 516. The control unit 515 is equipped with an intervention control unit 5151 and a measurement control unit 5152. The electrode 511 provides electrical muscle stimulation to the living body. The environmental thermometer 512, the body surface thermometer 513, and the body surface hardness tester 514 have the same configuration as the environmental thermometer 104, the body surface thermometer 108, and the body surface hardness tester 109 of the above embodiment.

The measurement control unit 5151 controls the intervention by electrical muscle stimulation of the intervention portion by controlling the electrodes 511. The measurement control unit measures the environmental temperature, body surface temperature, and body surface hardness by controlling the environmental thermometer 104, the body surface thermometer 108, and the body surface hardness tester 109, respectively. The communication unit 516 is a communication module that performs short-range wireless communication with the information terminal device 53.

The body composition analyzer 52 is a four-electrode BI measuring instrument equipped with four electrodes, including two current-carrying electrodes 521 and two measurement electrodes 522. The body composition analyzer 52 is a four-electrode BI measuring instrument equipped with four electrodes, including two current-carrying electrodes 521 and two measurement electrodes 522. In addition to the electrodes 521 and 522, the body composition analyzer 52 has a measurement control unit 523 and a communication unit 524. The measurement control unit 523 controls the current-carrying electrodes 521 to apply a weak current to the living body and measures the bioelectrical impedance by measuring the current at the measuring electrodes 522. The communication unit 524 is a communication module that performs short-range wireless communication with the information terminal device 53.

The information terminal device 53 has a touch panel 531, a control unit 532, and a communication unit 533. The touch panel 531 displays information and accepts touch input from the user. The control unit 532 operates according to a predetermined OS and performs various operations by executing application programs that run on the OS. The communication unit 533 has a function to perform short-range wireless communication with the EMS device 51 and the body composition analyzer 542 and a function to perform communication with the storage device 54 in the communication network via a router. The touch panel 531 corresponds to the combination of the display panel 101 and operation buttons 103 in the above embodiment.

In the above embodiment, the electrodes used to provide electrical stimulation to the living body in the EMS device, and the electrodes used to distribute electricity to the living body and measure it in the body composition analyzer are common electrodes, but in this variation, the EMS device 51 and body composition analyzer 52 are each equipped with electrodes. In the information terminal device 53, the control unit 532 executes the application program to control the intervention control unit 5151 and measurement control unit 5152 of the EMS device 51 via the communication unit 533 and communication unit 516 and control the measurement control unit 523 of the body composition meter 52 via the communication unit 533 and communication unit 524, in response to the user's input to the touch panel 531.

By executing the application program by the information terminal device 53, the control unit 532 functions as the output control unit 13, evaluation calculation unit 14, and the memory control unit 15 of the above embodiment. In this variant of the biometric measurement system 50, the touch panel 531, which corresponds to the display panel 101, is configured as a separate device from the EMS device 51 and the body composition analyzer 52, which are in contact with the body, so that the real-time response of the living body during the intervention can be easily confirmed visually.

The biometric measurement system 50 in this embodiment, since multiple EMS devices 51 will be installed in each part of the human body, the following controls will be performed in addition to the embodiment described above. In other words, the control unit 532 of the information terminal device 53 performs control for each EMS device 51 (i.e., for each intervention portion) based on the biometric information measured during the course of the intervention program. For example, the control unit 532 may determine the intervention portions based on the biometric information during the course of the intervention. In other words, the control unit 532 determines the content of the intervention, including the start and end of the intervention, for each EMS device 51 based on the measured biometric information. This makes it possible to control the dynamic change of the intervention content for each intervention portion based on the biometric information.

In this variation, the EMS device 51 as the intervention device and the body composition analyzer 52 as the biometric measurement device are provided as separate devices, and all biometric information is measured by the body composition analyzer 52, but in addition to the body composition analyzer 52, the EMS device 51 may also measure biometric information (e.g., bioelectrical impedance, body surface temperature, and body surface hardness).

### (Variant 2)

In the above embodiment, the intervention on the living body was to provide electrical stimulation that causes the muscles to perform reflexive contraction movements, but the intervention on the living body is not limited to this. The intervention to the living body may be, for example, to give thermal, mechanical, optical, electrical, chemical, or plasma stimulation to the living body. The purpose of the intervention may also be therapeutic, massage, relaxation, rehabilitation, etc., as well as training as described above.

In the biometric measurement system of Variant 2, the intervention device is a massager that gives mechanical stimulation to a living body to massage it as an intervention of physical stimulation to the living body. The massager is worn on the human body, which is a living body and is equipped with an air sac. The massager presses on the part of the human body to which it is attached by pumping air into the air sacs and releases the pressure by removing the air from the air sacs. The massager may also perform massage by having a member that performs pressing, striking, friction, etc., against the body surface operated by a power source such as a motor. The massager performs massage intervention by repeating such pressing and other massage actions. The fact that an intervention program is provided and various massage operations are performed in accordance with the intervention program, that various modes are provided, that the intensity can be adjusted, etc., is the same as the EMS device of the above form. This is the same as the EMS device in the above form.

The body composition values are stored in the control unit 532. The control unit 532 estimates the hardness of the normal body surface based on the amount of subcutaneous fat and the amount of muscle in the body composition values. The measurement control unit 5152 measures the hardness of the actual body surface of the living body using the body surface hardness tester 514. When there is stiffness in the living body, the muscles stiffen, and the body surface becomes hard. When a massage is performed, the stiffness is released, and the hardness of the body surface is reduced.

Therefore, the control unit 532 evaluates stiffness (fatigue) based on the stiffness of the body surface estimated from the body composition values, the stiffness of the body surface before the intervention, and the stiffness of the body surface after or during the intervention. The control unit 532 evaluates, for example, that the stiffness of the living body is relaxed based on the change from the stiffness of the body surface before the intervention to the stiffness of the body surface after or during the intervention, and that the stiffness of the living body has been eliminated when the stiffness of the body surface after or during the intervention becomes close to the normal surface stiffness estimated from the body composition.

At this time, the control unit 532 may also take into account the body surface temperature measured by the body surface thermometer 513 and the bioelectrical impedance measured by the body composition analyzer 513 to evaluate that the stiffness of the living body has been relaxed or that the stiffness has been eliminated. In other words, the bioelectrical impedance also changes as the body temperature rises and the blood circulation improves due to the effective massage. Therefore, the control unit 532 may evaluate the relaxation or elimination of stiffness as an effect of the massage, taking into account this biological information as well.

### (Variant 3)

In an intervention program, especially a training intervention program, a break may be taken in the middle of the program. For example, after having a user perform a passive exercise for about 10 minutes, the user may take a break for a few minutes and then perform the passive exercise for about 10 minutes again. Therefore, the biometric data may be repeatedly measured at predetermined time intervals (e.g., 30-second intervals) during the rest period. Then, based on the measured biometric information, it may be evaluated whether the fatigue of the living body is recovered, and when it is recovered, the next set of passive exercises may be started. The control unit that performs such control corresponds to the intervention start determination unit.

### (Variant 4)

In the above embodiment and its variants, the biometric information was measured after the intervention was performed using the intervention device. In particular, in the above embodiment, the EMS device as the intervention device made the living body perform a passive movement. In this variant, the biometric measurement system is not equipped with an intervention device, and the intervention contents are presented to the user to make the user perform an active exercise. In this way, the user applying a load to the muscles by his or her own will is also an intervention of physical stimulation to the muscles.

The biometric measurement system of this variation determines the intervention contents and informs the user of the determined intervention contents by displaying the intervention contents on a display panel or touch panel. At this time, the biometric measurement system guides the user through active exercise, which is the intervention content, and also guides the user through measurement using a body composition analyzer. The user performs the active exercise and measurement according to the displayed intervention contents. Specifically, the user follows the guidance to measure the biometric information before the intervention before the active exercise, then performs the active exercise, and measures the biometric information after the intervention when the guided content and amount of active exercise are completed. The biometric information may be measured in the middle of the active exercise if the active exercise can be performed while the biometric information can be measured.

The evaluation calculation unit evaluates the response of the living body to the active exercise based on the active exercise presented to the user, the biometric information before the intervention, and the biometric information during or after the intervention. The evaluation results are then presented to the user in association with the intervention and stored in a memory device. When the biometric information is measured during the intervention, the content of the subsequent active exercise may be adjusted according to the biometric information and presented to the user.

The intervention in this variant is not limited to exercise but may also be other dynamic exercises, massage, etc., in which the living body is stimulated by others.

### (Second embodiment)

In the first embodiment, the pad-like device that is the biometric measurement system 100 combines the functions of an intervention device (leg EMS) and a biometric measurement device, and the same electrodes 107 were used both as electrodes for the intervention device to provide electrical muscle stimulation to the living body and as electrodes for current-carrying and measuring the biometric measurement device. In addition, Patent No. 6367510 discloses an electrical muscle stimulator that uses electrodes of an electrical stimulation device to measure body composition, skin moisture, etc.

However, since the material of the electrode suitable for providing electrical muscle stimulation is different from the material of the electrode suitable for measuring bioelectrical impedance, both electrodes may be provided separately.

In this case, for example, as the electrode for electrical muscle stimulation, a material that can provide suitable electrical stimulation even if the contact impedance is relatively large may be selected, and as the electrode for bioelectrical impedance measurement, a material that can provide accurate measurement even if the contact impedance is relatively small may be selected. In addition, the number of electrodes for electrical muscle stimulation and the number of electrodes for bioelectrical impedance measurement may be different. In other words, two electrodes for electrical muscle stimulation are sufficient, but to perform the measurement or four-electrode BI method, four electrodes for bioelectrical impedance measurement may be used. In addition, the number of electrodes for electrical muscle stimulation may be more than two, and the number of electrodes for bioelectrical impedance measurement may be more than four.

Therefore, in this embodiment, the electrodes for electrical muscle stimulation and the electrodes for bioelectrical impedance measurement are separate electrodes. This makes it possible to adopt suitable materials for each electrode. In addition, it is possible to avoid the inconvenience that the contact impedance is too large, and the accurate measurement cannot be done when the biometric measurement is done with the electrode of the material suitable for the intervention of the electric stimulation. Moreover, it is possible to avoid the inconvenience that the user feels pain due to stimulation that is more than necessary for the living body by applying electrical stimulation with electrodes made of materials suitable for biometric measurement.

The biometric measurement system in this embodiment will be described below with reference to the drawings. In the following description, electrodes for electrical muscle stimulation and electrodes for bioelectrical impedance measurement will be described mainly. For other configurations of the biometric measurement system, the first embodiment can be applied.

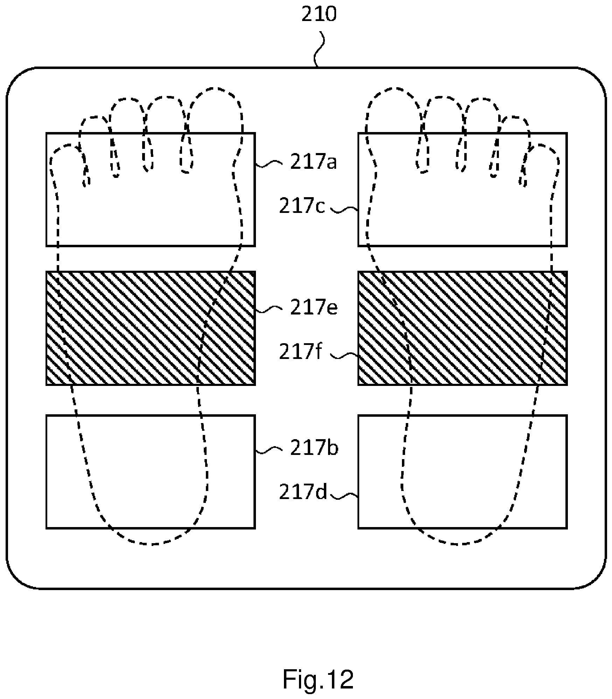

Fig. 12 is an external view of the biometric measurement system of this embodiment. The biometric measurement system 210 has a platelike shape and intervenes electrical stimulation, and measures bioelectrical impedance by placing both feet on it and making contact with both soles. The electrodes for bioelectrical impedance measurement and the electrodes for electrical muscle stimulation are arranged in such a way that the user can receive the biometric measurement and the intervention of electrical muscle stimulation without changing the body position. The user can also receive the electrical stimulation intervention and the bioelectrical impedance measurement in both standing and sitting positions. The electrode for bioelectrical impedance measurement and the electrode for electrical muscle stimulation are separated from each other but are arranged in such a way that they can contact the same part of the living body (in this embodiment, the sole of the foot) at the same time.

A left current-carrying electrode 217a, a left measuring electrode 217b, a right current-carrying electrode 217c, and a right measuring electrode 217d which respectively contact the anterior (base of the fingers) and posterior (heel) portions of the left sole and the anterior (base of the fingers) and posterior (heel) portions of the right sole, as electrodes for bioelectrical impedance measurement, are provided on the upper surface of the biometric measurement system 210. A left electrical muscle stimulation electrode 217e and a right electrical muscle stimulation electrode 217f are provided on the center of the left sole (arch part of the foot) and the center of the right sole (arch part of the foot) of the upper surface, respectively. In this way, the electrodes 217a-217d for bioelectrical impedance measurement and the electrodes 217e and 217f for electrical muscle stimulation are provided adjacent to each other.

Surfaces of the left electrical muscle stimulation electrode 217e and the right electrical muscle stimulation electrode 217f may be higher than those of the electrodes 217a to 217d for bioelectrical impedance measurement, for example, by 3 to 50 mm. In this case, the left electrical muscle stimulation electrode 217e and the right electrical muscle stimulation electrode 217f may have a shape raised so as to fit the shape of the arch of the foot. Or, the left electrical muscle stimulation electrode 217e and the right electrical muscle stimulation electrode 217f may have a damper member such as a spring and thereby can be pushed in. These configurations allow the left electrical muscle stimulation electrode 217e and the right electrical muscle stimulation electrode 217f to fit the sole of the foot in a suitable manner to provide electrical stimulation to the living body in an appropriate manner.

For both the electrodes for bioelectrical impedance measurement and the electrodes for electrical muscle stimulation, the state of contact between the electrodes and the living body (sole of the foot) can be confirmed by using a system to check the continuity. For example, a system that calculates the Cole circle of the measurement data using the resistance and reactance in bioelectrical impedance and determines whether there is an abnormal measurement of bioimpedance based on the amount of deviation from the normal Cole circle can be employed as such a system (See, for example, JP2019-84156). Alternatively, a system that determines the cause and type of measurement abnormality based on time-series data of bioelectrical impedance can be employed as such a system.

Separate circuits for control and measurement may be provided for electrodes for electrical muscle stimulation and electrodes for bioelectrical impedance measurement. Alternatively, the circuitry may be common, and each of the six electrodes may be switched for electrical muscle stimulation and bioelectrical measurement according to the situation.

Although the above embodiments describe electrical stimulation intervention and electrical impedance measurement using electrodes, the biometric measurement system 210 may be accompanied by a height meter and a weight meter. For example, the leg EMS may be set at an angle to the floor in order to make it easier to place the legs in a sitting position, in this case, a spring or damper with a force that does not interfere with the leg movement by the leg EMS can be builtin, so that four points (at least three points) naturally touch the top surface in a standing position for weight measurement.

In the above embodiments, the electrodes for bioelectrical impedance measurement and electrical muscle stimulation are placed in close proximity to each other, but the electrodes for bioelectrical impedance measurement and electrical muscle stimulation can be swapped internally by a sliding or rotating mechanism, and both types of electrodes can be placed in the same position.

In addition, the above embodiments describe a biometric measurement system in which the electrodes are in contact with the soles of the feet, but the system may also be used to contact other parts of the body, such as the hands, for example.

The above embodiments describe a biometric measurement system in which the electrode for bioelectrical impedance measurement and the electrode for electrical muscle stimulation are separately installed with reference to Fig. 12, but the example in which electrodes for bioelectrical impedance measurement and electrodes for electrical muscle stimulation are separately installed is not limited to the configuration shown in Fig. 12. In the following, variants of the arrangement of electrodes for bioelectrical impedance measurement and electrodes for electrical muscle stimulation are explained.

In the following variation, the electrodes are placed in one of four locations on the top surface: top left (corresponding to the left toe side), top right (corresponding to the right toe side), bottom left (corresponding to the left heel side), and bottom right (corresponding to the right heel side). Since the toe and heel sides of the foot are sure to be grounded in both standing and sitting positions, while the center of the foot is slightly indented, the placement of the electrodes at any of these four locations ensures reliable contact between the body and the electrodes.

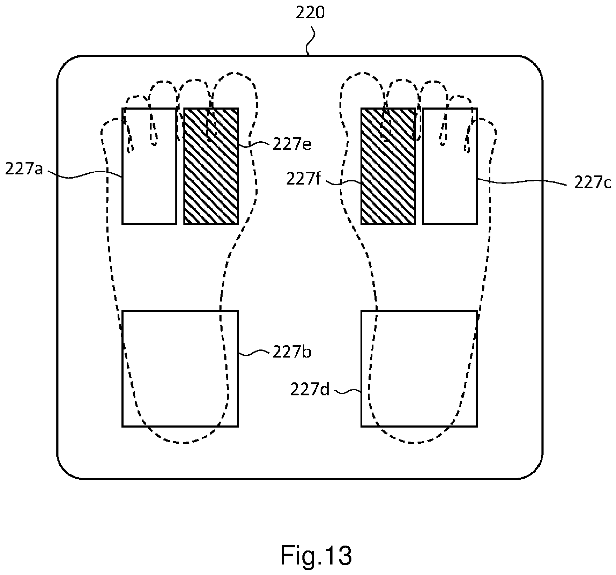

Fig. 13 shows a first variation of the electrode arrangement. In the biometric measurement system 220 of the first variation, in the upper left position and upper right position, the electrodes for bioelectrical impedance measurement 227a, 227c are placed on the outer side, and electrodes for electrical muscle stimulation 227e and 227f are placed on the inner side. The electrodes for bioelectrical impedance measurement 227b and 227d are placed at the lower left and lower right positions, respectively.

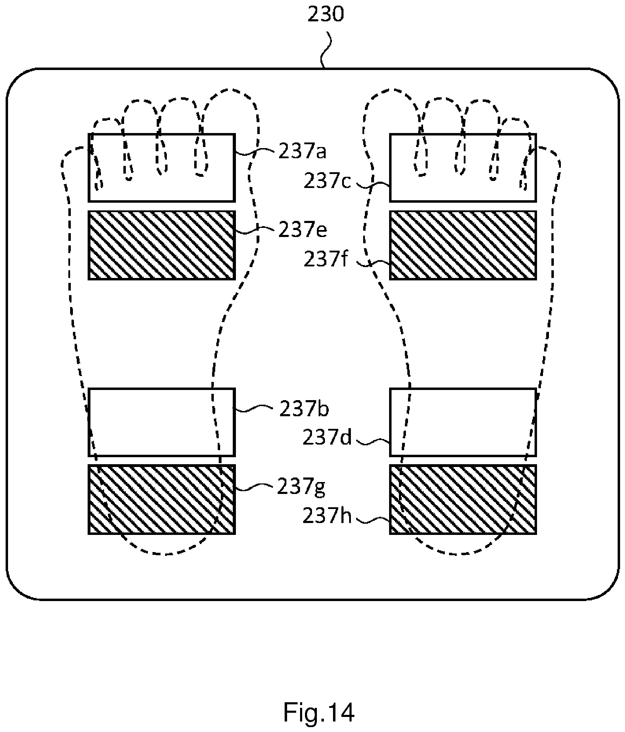

Fig. 14 shows the second variation of the electrode arrangement. In the biometric measurement system 230 of the second variation, respectively in the upper left position, upper right position, lower left position, and lower right position, the electrodes for bioelectrical impedance measurement 237a, 237c, 237b, and 237d are placed on the upper side (toe side), and the electrodes for electrical muscle stimulation 237e, 237f, 237g, 237h are placed on the lower side (heel side). In this example, the distance in front-back direction between the electrodes for bioelectrical impedance measurement and the distance in front-back direction between the electrodes for electrical muscle stimulation are the same, and even a user with small feet can ensure that the four electrodes contact the foot by changing the position of the foot between the time of bioelectrical measurement and the time of electrical stimulation intervention.

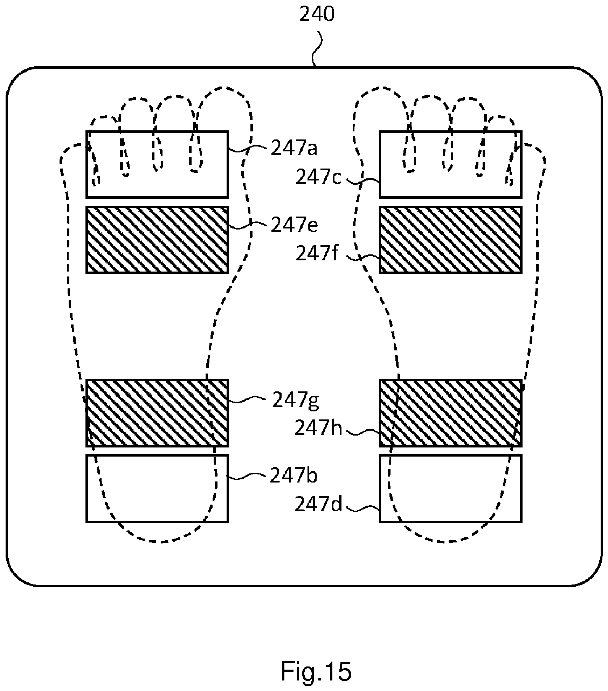

Fig. 15 shows a third variation of the electrode arrangement. In the biometric measurement system 240 of the third variation, respectively in the upper left position, upper right position, loser left position, and lower right position, the electrodes for bioelectrical impedance measurement 247e, 247f, 247g, and 247h are placed on the inner side of the front-back direction, and the electrodes for bioelectrical impedance measurement 247a, 247c, 247b, 247d are placed on the outer side of the front-back direction. In this example, the electrodes for bioelectrical impedance measurement are sufficiently far apart in the front-back direction to increase the accuracy of biometric measurement.

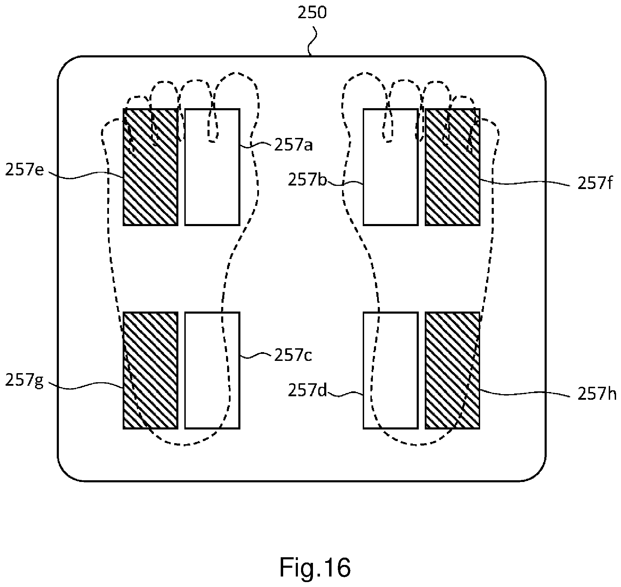

Fig. 16 shows a fourth variation of the electrode arrangement. In the biometric measurement system 250 of the fourth variation, respectively in the upper left position, upper right position, lower left position, and lower right position, the electrodes for bioelectrical impedance measurement 257a, 257c, 257b, 257d are placed on the inner side in the left-right direction, and electrodes for electrical muscle stimulation 257e, 257f, 257g, and 257h are placed on the outer side in the left-right direction. In this example, the bioelectrical impedance measurement electrodes and electrical muscle stimulation electrodes are arranged in the left-right direction, so the bioelectrical impedance measurement electrodes and electrical muscle stimulation electrodes which are longer in the front-back direction than in the examples shown in Figs. 12 to 15 can be used. Therefore, the distance between the electrodes for bioelectrical impedance measurement in the front-back direction and the distance between the electrodes for electrical muscle stimulation in the front-back direction can be shortened, and the four electrodes can be made to contact the foot without fail, even for a user with small feet.

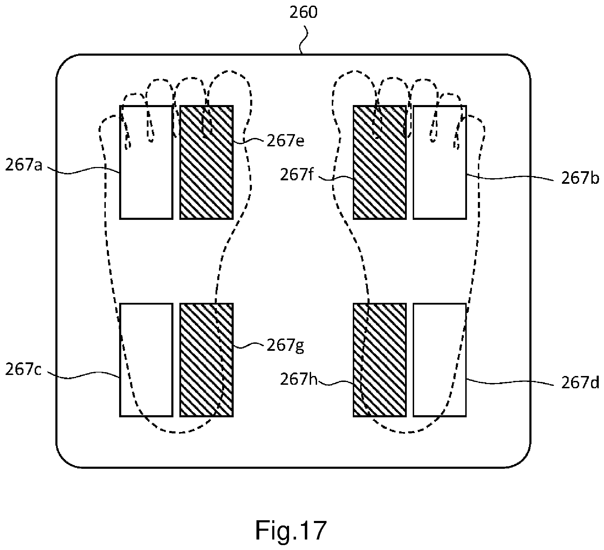

Fig. 17 shows a fifth variation of the electrode arrangement. In the biometric measurement system 260 of the fifth variation, respectively in the upper left position, upper right position, lower left position, and lower right position, the electrodes for electrical muscle stimulation 267e, 267f, 267g, and 267h are placed on the inner side in the front-back direction, and the electrodes for bioelectrical impedance measurement 267a, 267c, 267b, 267d are placed on the outer side in the front-back direction. In this example, the distance between the front and back of the electrodes for bioelectrical impedance measurement and the front and back of the electrodes for electrical muscle stimulation are the same, and even a person with small feet can ensure that the four electrodes contact the feet. In addition, since the distance of the electrodes for bioelectrical impedance measurement in the left-right direction does not affect the measurement accuracy, this example also has the same effect as the fourth example shown in Fig. 16.

The above description discloses the following biometric measurement system and biometric measurement program.

A biometric measurement system in one aspect comprises: an intervention determination unit for determining contents of intervention of physical stimulation to a living body; a biometric measuring unit for measuring biometric information of the living body; and an output unit that outputs the biometric information measured by the biometric measurement unit or information based on the biometric information associated with the intervention determined by the intervention determination unit.

This configuration makes it possible to know the immediate response of the living body to the intervention of a physical stimulus to the living body. The physical stimulus may be, for example, thermal stimulation, mechanical stimulation, optical stimulation, electrical stimulation, chemical stimulation, and plasma stimulation.

In the biometric measurement system described above, the biometric measurement unit may measure bioelectrical impedance, body surface temperature, and/or body surface hardness of the living body as the biometric information.

With this configuration, bioelectrical impedance, body surface temperature, and/or body surface hardness, or information based thereon, can be obtained as a response of the living body due to the intervention of a physical stimulus to the living body.

The biometric measurement system described above may further comprise an intervention device that intervenes with a physical stimulus to the living body according to the contents of intervention determined by the intervention determination unit.

This configuration allows the intervention device to intervene with physical stimulus to the living body and to know the immediate response of the living body to the intervention.

In the biometric measurement system described above, the intervention device may apply, as the intervenes with a physical stimulus, an electrical stimulus that reflexively contracts muscles of the living body.

With this configuration, it is possible to know the reaction of the living body when the muscle contraction exercise is performed as a passive exercise by applying the muscle an electrical stimulus, that is, the effect of the passive exercise by the intervention device. The device that applies electrical stimulation to reflexively contract the muscles of a living body is typically an EMS device.

In the biometric measurement system described above, the intervention device may apply the electrical stimulation to the living body using an electrode, and the biometric measuring unit may measure the bioelectrical impedance of the living body as the biometric information using the electrode.

With this configuration, the electrode for applying the electrical stimulation for passive exercise can also be used as the electrode for bioelectrical impedance measurement.

In the biometric measurement system described above, the intervention device may apply the electrical stimulation to the living body using an electrode, and the biometric measuring unit may measure the bioimpedance of the living body as the biometric information using an electrode different from the electrode of the intervention device.

With this configuration, the electrodes for applying electrical stimulation for passive exercise and the measurement electrodes bioelectrical impedance can be different electrodes.

In the biometric measurement system described above, the intervention device may apply a mechanical stimulus to the living body as the intervention with a physical stimulus.

This configuration allows the user to know the response of the living body to an intervention (e.g., massage) that applies a physical stimulus to muscles or skin, i.e., the effect of a massage or the like by the intervention device.

In the biometric measurement system described above, the biometric measuring unit may measure, as the biometric information, pre-intervention biometric information obtained before the intervention is performed on the living body and in-process biometric information and/or post-intervention biometric information obtained when the intervention is performed on the living body, and the output unit may output a change from the pre-intervention biometric information to the in-process biometric information and/or the post-intervention biometric information or information based on the change.

This configuration allows the user to know the response of the living body to the physical stimulus by the change in the biometric information after the start of the intervention of the physical stimulus to the living body.

The biometric measurement system described above may further comprise an informing device that informs the contents of the intervention determined by the intervention determination unit.

With this configuration, for example, when a predetermined training menu or massage menu is determined as the content of physical stimulation, the informing unit informs the user of the training menu or massage menu so that the user can perform training or massage according to the informed training menu or massage menu. The biometric measurement unit can measure the biometric information of the user who performs such training and massage.

In the biometric measurement system described above, the intervention determination unit may determine the contents of the intervention according to a user operation.

This configuration allows the user to know the response of the living body to the intervention content determined by the user.

A biometric measurement system in one aspect comprises: an intervention device for intervening with a physical stimulus to a living body; and a biometric measurement unit that measures biometric information of the living body during the intervention by the intervention device.

This configuration allows the biometric information in the middle of the intervention to be obtained.

In the biometric measurement system described above, the intervention device may determine the subsequent content of the intervention based on the biometric information.

This configuration allows the subsequent intervention content to be dynamically determined based on the real-time response of the living body to the intervention of the physical stimulus. Determining the content of the subsequent intervention includes stopping the intervention. For example, the intervention by the intervention device can be controlled to continue until the effect of the intervention is sufficient, and then the intervention by the intervention device can be stopped when the effect of the intervention is sufficient.

In the biometric measurement system described above, the intervention device may be a device that performs said intervention on a plurality of intervention portions of said biological body, and the biometric measurement system may further comprise an intervention determination unit that determines the content of the subsequent intervention for each of the intervention portions based on the biometric information.

This configuration allows the intervention content to be determined for each intervention portion based on the biometric information.

A biometric measurement system in one aspect comprises: an intervention device that intervenes with a physical stimulus to a living body; a biometric measuring unit that measures biometric information of a living body; and an intervention start determination unit that determines the timing of the start of the next intervention by the intervention device on the living body, based on the biometric information measured by the biometric measurement unit after the end of the intervention.

With this configuration, the start timing of the next intervention can be determined based on the biometric information after the intervention is completed.

A biometric measurement system in one aspect comprises: an intervention determination unit that determines contents of intervention of physical stimulation to a living body; a body composition calculating unit that calculates a body composition of the living body; and a memory unit that stores the body composition and the contents of the intervention in association with each other.

With this configuration, since the content of the physical stimulus and the body composition at the time the physical stimulus is given are stored in association with each other, by repeating this storage over a certain period of time, the change or transition of the body composition due to the physical stimulus in that period of time can be known.

A biometric measurement program in one aspect makes a computer function as: an intervention determination unit for determining contents of intervention of physical stimulation to a living body; a biometric measuring unit for measuring biometric information of the living body; and an output unit that outputs the biometric information measured by the biometric measurement unit or information based on the biometric information associated with the intervention determined by the intervention determination unit.

A biometric measurement program in one aspect makes a computer function as: an intervention device for intervening with a physical stimulus to a living body; and a biometric measurement unit that measures biometric information of the living body during the intervention by the intervention device.

A biometric measurement program in one aspect makes a computer function as: an intervention device that intervenes with a physical stimulus to a living body; a biometric measuring unit that measures biometric information of a living body; and an intervention start determination unit that determines the timing of the start of the next intervention by the intervention device on the living body, based on the biometric information measured by the biometric measurement unit after the end of the intervention.

A biometric measurement program in one aspect makes a computer function as: an intervention determination unit that determines contents of intervention of physical stimulation to a living body; a body composition calculating unit that calculates a body composition of the living body; and a memory unit that stores the body composition and the contents of the intervention in association with each other.

### DESCRIPTION OF THE CODE

10...Control unit
11... Intervention control unit
12...Measurement control unit
13...Output control unit
14...Evaluation calculation unit
15...Memory control unit
20...Storage device
100, 210, 220, 230, 240, 250, 260...Biometric measurement system
101...Display panel
102...Start button
103...Operation button
104...Environmental thermometer
107...Electrodes
108...Body Surface Thermometer
109...Body surface hardness tester
217a to 217d, 227a to 227d, 237a to 237d, 247a to 247d, 257a to 257d, 267a to 267d...Electrodes for bioelectrical impedance measurement
217e to 217f, 227e to 227f, 237e to 237h, 247e to 247h, 257e to 257h, 267e to 267h...Electrodes for electrical muscle stimulation

## Claims

1. A biometric measurement system capable of providing electrical muscle stimulation to a living body, comprising:
an electrode for bioelectrical impedance measurement for measuring a bioelectrical impedance of a living body, and
an electrode for electrical muscle stimulation for applying electrical muscle stimulation to the living body, wherein
the bioelectrical impedance measurement electrode and the electrical muscle stimulation electrode are arranged to contact the living body simultaneously.

2. The biometric measurement system according to claim 1, wherein the electrode for bioelectrical impedance measurement and the electrode for electrical muscle stimulation are disposed apart from each other.

3. The biometric measurement system according to claim 2, wherein the electrode for bioelectrical impedance measurement and the electrode for electrical muscle stimulation are arranged to contact a sole of a foot of the living body simultaneously.

4. The biometric measurement system according to any one of claims 1 to 3, comprising:
a plurality of said electrodes for bioelectrical impedance measurement for a left portion;
a plurality of said electrodes for bioelectrical impedance measurement electrodes for a right portion;
at least one said electrode for electrical muscle stimulation for a left portion; and
at least one said electrode for electrical muscle stimulation for a right portion.

5. The biometric measurement system according to claim 4, comprising:
the electrode for bioelectrical impedance measurement of the toe side for the left foot;
the electrode for bioelectrical impedance measurement of the heel side for the left foot;
the electrode for electrical muscle stimulation of a central portion for the left foot;
the electrode for bioelectrical impedance measurement of the toe side for the right foot;
the electrode for bioelectrical impedance measurement of the heel side for the right foot; and
the electrode for electrical muscle stimulation of a central portion for the right foot.

6. The biometric measurement system according to claim 5, wherein the electrodes for electrical muscle stimulation have a raised shape that fits the shape of the central portion.

7. The biometric measurement system according to claim 5, wherein the electrodes for electrical muscle stimulation are raised and can be pushed in.

8. The biometric measurement system according to claim 4, comprising:
the electrode for bioelectrical impedance measurement of the toe side for the left foot;
the electrode for bioelectrical impedance measurement of the heel side for the left foot;
the electrode for electrical muscle stimulation of the toe side for the left foot;
the electrode for electrical muscle stimulation of the heel side for the left foot;
the electrode for bioelectrical impedance measurement of the toe side for the right foot;
the electrode for bioelectrical impedance measurement of the heel side for the right foot; and
the electrode for electrical muscle stimulation of the toe side for the right foot; and
the electrode for electrical muscle stimulation on the heel side for the right foot.

9. The biometric measurement system according to any one of claims 1 to 8, wherein the electrode for bioelectrical impedance measurement and the electrode for electrical muscle stimulation are made of different materials.

10. The biometric measurement system according to any one of claims 1 to 9, further comprising an output unit that outputs information based on the bioelectrical impedance measured using the electrode for the bioelectrical impedance measurement in association with the intervention by the electrical muscle stimulation using the electrodes for electrical muscle stimulation.

11. A biometric measurement system, comprising:
an intervention determination unit for determining contents of intervention of physical stimulation to a living body;
a biometric measuring unit for measuring biometric information of the living body; and
an output unit that outputs the biometric information measured by the biometric measurement unit or information based on the biometric information associated with the intervention determined by the intervention determination unit.

12. The biometric measurement system according to claim 11, wherein the biometric measurement unit measures bioelectrical impedance, body surface temperature, and/or body surface hardness of the living body as the biometric information.

13. The biometric measurement system according to claim 11 or 12, further comprising an intervention device that intervenes with a physical stimulus to the living body according to the contents of intervention determined by the intervention determination unit.

14. The biometric measurement system according to claim 13, wherein the intervention device applies, as the intervenes with a physical stimulus, an electrical stimulus that reflexively contracts muscles of the living body.

15. The biometric measurement system according to claim 14, wherein the intervention device applies the electrical stimulation to the living body using an electrode, and
the biometric measuring unit measures the bioelectrical impedance of the living body as the biometric information using the electrode.

16. The biometric measurement system according to claim 14, wherein
the intervention device applies the electrical stimulation to the living body using an electrode, and
the biometric measuring unit measures the bioimpedance of the living body as the biometric information using an electrode different from the electrode of the intervention device.

17. The biometric measurement system according to claim 13, wherein the intervention device applies a mechanical stimulus to the living body as the intervention with physical stimulus.

18. The biometric measurement system according to claim 11, wherein
the biometric measuring unit measures, as the biometric information, pre-intervention biometric information obtained before the intervention is performed on the living body and in-process biometric information and/or post-intervention biometric information obtained when the intervention is performed on the living body, and
the output unit outputs a change from the pre-intervention biometric information to the in-process biometric information and/or the post-intervention biometric information or information based on the change.

19. The biometric measurement system according to claim 11, further comprising an informing device that informs the contents of the intervention determined by the intervention determination unit.

20. The biometric measurement system according to claim 11, wherein the intervention determination unit determines the contents of the intervention according to a user operation.

21. A biometric measurement system, comprising:
an intervention device for intervening with a physical stimulus to a living body; and
a biometric measurement unit that measures biometric information of the living body during the intervention by the intervention device.

22. The biometric measurement system according to claim 21, wherein the intervention device determines the subsequent content of the intervention based on the biometric information.

23. The biometric measurement system according to claim 21, wherein
the intervention device is a device that performs said intervention on a plurality of intervention portions of said biological body, and
the biometric measurement system further comprises an intervention determination unit that determines the content of the subsequent intervention for each of the intervention portions based on the biometric information.

24. A biometric measurement system, comprising:
an intervention device that intervenes with a physical stimulus to a living body;
a biometric measuring unit that measures biometric information of a living body; and
an intervention start determination unit that determines the timing of the start of the next intervention by the intervention device on the living body, based on the biometric information measured by the biometric measurement unit after the end of the intervention.

25. A biometric measurement system, comprising
an intervention determination unit that determines contents of intervention of physical stimulation to a living body;
a body composition calculating unit that calculates a body composition of the living body; and
a memory unit that stores the body composition and the contents of the intervention in association with each other.

26. A biometric measurement program that makes a computer function as:
an intervention determination unit for determining contents of intervention of physical stimulation to a living body;
a biometric measuring unit for measuring biometric information of the living body; and
an output unit that outputs the biometric information measured by the biometric measurement unit or information based on the biometric information associated with the intervention determined by the intervention determination unit.

27. A biometric measurement program that makes a computer function as:
an intervention device for intervening with a physical stimulus to a living body; and
a biometric measurement unit that measures biometric information of the living body during the intervention by the intervention device.

28. A biometric measurement program that makes a computer function as:
an intervention device that intervenes with a physical stimulus to a living body;
a biometric measuring unit that measures biometric information of a living body; and
an intervention start determination unit that determines the timing of start of the next intervention by the intervention device on the living body, based on the biometric information measured by the biometric measurement unit after the end of the intervention.

29. A biometric measurement program that makes a computer function as:
an intervention determination unit that determines contents of intervention of physical stimulation to a living body;
a body composition calculating unit that calculates a body composition of the living body; and
a memory unit that stores the body composition and the contents of the intervention in association with each other.

30. A computer-readable non-transitory storage medium on which is recorded the biometric measurement program according to any of claims 26 to 29.
